# EUROPEAN PATENT SPECIFICATION

(11) **EP 3 788 116 B1**
(45) Date of publication and mention of the grant of the patent: **07.09.2022**
(21) Application number: 19730939.6
(22) Date of filing: 03.05.2019
(51) Int. Cl.: C09K 11/00, C07D 487/04

(54) **CHEMILUMINESCENT IMIDAZOPYRAZINONE-BASED PHOTOSENSITIZERS WITH AVAILABLE SINGLET AND TRIPLET EXCITED STATES**
CHEMILUMINESZIERENDE IMIDAZOPYRAZINONBASIERTE FOTOSENSIBILISATOREN MIT VERFÜGBAREN ANGEREGTEN SINGLETT- UND TRIPLETT-ZUSTÄNDEN
PHOTOSENSIBILISATEURS CHIMIOLUMINESCENTS À BASE D'IMIDAZOPYRAZINONE, PRÉSENTANT DES ÉTATS EXCITÉS SINGULETS ET TRIPLETS DISPONIBLES

(30) Priority: 04.05.2018 PT 2018110719
(43) Date of publication of application: 10.03.2021
(73) Proprietor: Universidade do Porto, 4099-002 Porto (PT)
(72) Inventor: GOMES ESTEVES DA SILVA, Joaquim Carlos, 4169-007 Porto (PT); DA COSTA PINTO DA SILVA, Luís Tiago, 4250-287 Porto (PT); RODRIGUEZ BORGES, Jose Enrique, 4169-007 Porto (PT); NÚÑEZ MONTENEGRO, Ara, 4100-450 Porto (PT); ANTUNES CRISTA, Diana Maria, 4400-045 Vila Nova De Gaia (PT); OLIVEIRA FERREIRA, Paulo José, 4455-513 Perafita (PT)
(74) Representative: Ferreira, Maria Silvina
(86) International application number: PCT/IB2019/053642
(87) International publication number: WO 2019/211809

(56) References cited:
- WO-A1-2015/075483
- CN-A- 105 968 114
- JP-A- H11 209 379
- JP-A- 2007 277 097
- US-A1- 2018 072 781
- CHIA-YEN HSU ET AL: "Bioluminescence resonance energy transfer using luciferase-immobilized quantum dots for self-illuminated photodynamic therapy", BIOMATERIALS, vol. 34, no. 4, 1 January 2013 (2013-01-01), pages 1204-1212, XP055126530, ISSN: 0142-9612, DOI: 10.1016/j.biomaterials.2012.08.044 cited in the application

## Description

### Technical field

This application relates to a self-activated chemiluminescent photosensitizer, the method to produce said photosensitizer and uses thereof.

### Background art

Currently, commercial photosensitizers used in clinical photodynamic therapy require activation by light irradiation by light/laser sources external to the body of the patient. However, typical photosensitizers strongly absorb UV or visible light, which is unsuitable for biological penetration due to light absorption and scattering by tissue. Moreover, the depth of light penetration into the tissues is less than 1 mm. Thus, photodynamic therapy is only used on tumors or just under the skin or on the outer lining of internal organs/cavities. Moreover, photodynamic therapy is also ineffective against metastatic tumors, given the localized nature of the used excitation sources.

Some research efforts have been devoted to the development of new types of excitation sources of deep-penetration photodynamic therapy. One of those strategies is to excite the photosensitizer with near-infrared light. However, this approach requires very expensive, energy-consuming and complex femtosecond pulsed lasers. Infrared upconversion nanoparticles based on rare earth metals have also been tested. In this case, these nanoparticles are excited with near-infrared systems, and then transfer their energy to the photosensitizer. Nevertheless, the use of metal ions decreases the biocompatibility and increase the toxicity of this system, thereby preventing its use in a clinical environment. X-rays, which have an unlimited depth of penetration into the body, have also been used as excitation source in photodynamic therapy. However, X-rays cannot activate the photosensitizer directly, and require the presence of X-ray-excitable nanoparticles that can transfer their energy to the photosensitizer. Also, X-ray radiation must be used many times to obtain a satisfactory cytotoxicity effect, which given the higher energy of this type of radiation, leads to significant damage to surrounding normal tissues.

Finally, some authors have used chemiluminescent and bioluminescent reactions to excite the photosensitizer by energy transfer. However, it is nearly impossible to ensure that the photosensitizer, the chemi-/bioluminescent substrates, co-factors and catalysts are also delivered selectively to tumor cells at the same time and to the same localization, without reacting during the delivery process. Moreover, all processes involving energy transfer are prone to efficiency losses, which diminishes the therapeutic potential. Also, none of these strategies possess tumorselectivity by itself, which eliminates the major advantages of conventional photodynamic therapy: its selectivity.

Photocatalysis has found widespread application in the fields of water splitting, carbon dioxide reduction, solar cell materials, air/water purification, and in organic synthesis. This field consists mainly on the use of photonic energy to target a light-absorbing compound (the photocatalyst), which upon photo-excitation can induce an accompanying substrate or reagent to participate in a reaction pathway not accessible under thermal control. This takes advantage of the known reactivity of electronically excited states. The photocatalysis-induced conversion of light into chemical energy in a selective manner includes two main mechanisms, irrespective of the nature of the catalyst (transition metal-based or fully organic). One of them is by energy transfer, in which the relaxation of the catalyst is coupled to the excitation of the substrate. More often, the energy transfer step occurs from the long-lived triplet state of the catalyst. Despite the advantages provided by photocatalysis, there are still several drawbacks associated with this approach. Most organic molecules are colorless and can be photoactivated by highenergy UV light, which is sufficiently reactive that can cause the uncontrolled fragmentation of many bonds in complex molecules. Moreover, as intersystem crossing is a spinforbidden process, it is difficult to develop organic photocatalysts that can cross to long-lived triplet states, which can be involved in energy transfer steps. Metal-based compounds are known for higher intersystem crossing rates, but present problems regarding toxicity, sustainability and cost. Furthermore, both organic and metal-based photocatalysts require energy-consuming equipment, which increases the cost and complexity of the process.

Document NO842089 (A) describes the synthesis of a purified hematoporphyrin derivative for diagnosis and treatment of tumors, and method. The tumor treatment properties result from the ability of photosensitizing the production of singlet oxygen. This invention requires the use of external light sources to trigger the activation of the hematoporphyrin derivative inside tumor cells, which limits the use of this photosensitizer on tumors or just under the skin or on the outer lining of internal organs/cavities. This dependence for external light sources also prevents this invention from being used on metastasis. The requirement for external light sources also increases the cost and complexity of the therapy process.

The referred invention also indicates the possibility of using the proposed hematoporphyrin derivative in tumor diagnostics, due to its ability to emit red light upon photo-excitation. However, the photo-excitation requires light irradiation by an external light source. This increases the probability for autofluorescence and for the increase of background noise, which decreases the sensitivity of the diagnostics. Moreover, the requirement of an external light source also limits the diagnostic to tumors or just under the skin or on the outer lining of internal organs/cavities.

D. Mao, W.B. Wu, S.L. Ji, C. Chen, F. Hu, D.L. Kong, D. Ding, Liu, B. Chemiluminescence-Guided Cancer Therapy Using a Chemiexcited Photosensitizer. Chem 2017, 3, 991-1007: describes the development of a novel nanomaterial with chemiexcited far-red/near-infrared emission and singlet oxygen generation for the treatment of tumors. This nanomaterial consists on bis[2,4,5-trichloro-6-(pentyloxycarbonyl)phenyl] oxalate (CPPO) as the chemiluminescence substrate, and an organic fluorogen with aggregation-induced emission as the photosensitizer. These compounds were co-encapsulated by pluronic F-127 and soybean oil to form the nanoparticles. CPPO emits light due to a chemiluminescence reaction with hydrogen peroxide (which is overexpressed in tumors), and acts as the excitation source of the photosensitizer. However, this approach is dependent on energy transfer steps, which are prone to efficiency losses, leading to diminished therapeutic potential. Moreover, this approach is based on many sequential steps (chemiluminescence -> energy transfer from the chemiluminophore to the photosensitizer -> intersystem crossing -> photosensitizer production of singlet oxygen), which increases the probability for efficiency losses along the therapeutic process. Furthermore, as energy transfer is inversely proportional to the distance between the acceptor and the donor, the proposed approach is not efficient if the nanocarrier (composed by pluronic F-127) is degraded before the occurrence of the chemiluminescent reaction, as the donor and acceptor would diffuse away from each other.

C.Y. Hsu, C.W. Chen, H.P. Yu, Y.F. Lin, P.S. Lai. Bioluminescence resonance energy transfer using luciferase-immobilized quantum dots for self-illuminated photodynamic therapy. Biomaterials 2013, 34, 1204-1212:
describes the development of Renilla luciferase-immobilized quantum dots (QD-RLuc8 conjugates) for bioluminescence resonance energy transfer-mediated photodynamic therapy of cancer. The QD-RLuc8 conjugates exhibit self-illumination at 655 nm after coelenterazine (the bioluminescent substrate) addition. This occurs due to the luciferase enzyme catalyzing the bioluminescence of coelenterazine, which transfers its energy to the quantum dots. These nanoparticles can then activate the photosensitizer Foscan^{®} by another energy transfer step. This approach has several drawbacks. First, it consists of two sequential energy transfer steps (from the bioluminophore to the quantum dots, and from the quantum dots to the photosensitizer), which should lead to significant efficiency losses. Second, the bioluminescent reaction here used (the Renilla luciferase-coelenterazine system) has no intrinsic tumor-specificity, and so, this therapeutic process has no selectivity for cancer. Finally, this approach requires three components (QD-RLuc8 conjugates, the photosensitizer Foscan^{®}, and coelenterazine) to be selectively delivered at the same time and to the same cellular localization without reacting during the delivery process, which is nearly impossible. In fact, the authors of this paper did not provide an answer for this problem.

F.J. Ai, N. Wang, X.M. Zhang, T.Y. Sun, Q. Zhu, W. Kong, F. Wang, G.Y. Zhu. An upconversion nanoplatform with extracellular pH-driven tumor-targeting ability for improved photodynamic therapy. Nanoscale 2018, 10, 4432-4441: describes an upconversion nanoplatform with extracellular pH-driven tumor-targeting ability for photodynamic therapy. This nanomaterial is composed by upconversion nanoparticles that can be photoexcited with near-infrared light, and which can activate the photosensitizer by energy transfer. The nanoplatform also includes the pH low insertion peptide (pHLIP), which can bring cargo specifically into cancer cells under an acidic environment. The proposed approach has advantages over conventional photodynamic therapy, as the near-infrared light used to activate the upconversion nanoparticles is more suitable for biological applications than the visible light generally used. Nevertheless, this type of approach also has several drawbacks. First, it is based on energy transfer steps, which are prone to efficiency losses. Moreover, while the near-infrared light used here is more suitable for biological applications, it requires highcost and complex femtosecond- or nanosecond-pulsed lasers. Finally, upconversion nanoparticles are composed of rare earth metals, which increases the cost and sustainability of the therapeutic process, while putting in question its biocompatibility.

5. S. Xu, P. Zhou, Z. Zhang, C. Yang, B. Zhang, K. Deng, S. Bottle, H. Zhu, Selective oxidation of 5-Hydroxymethylfurfural to 2,5-furandicarboxylic acid using O2 and a photocatalyst of Co-thioporphyrazine bonded to g-C3N4. Journal of the American Chemical Society 2017, 139, 14775-14782: describes the selective oxidation of 5-hydroxymethylfurfural to 2,5-furandicarboxylic acid under mild conditions by employing a photocatalyst, namely cobalt thioporphyrazine dispersed on g-C3N4. The oxidation reaction is driven by singlet oxygen, which is produced by cobalt thioporphyrazine. The proposed photocatalytic mechanism has the disadvantage of needing metal elements (cobalt) to induce the production of singlet oxygen, which increases the cost and potential toxicity of this approach while decreasing its sustainability. Moreover, this process requires energy-consuming light sources to activate the photocatalyst. Also, the used light source emits light in the UV region, which can lead to the uncontrolled fragmentation of unwanted bonds.

Document CN105968114 discloses a class of coelenterazine analogs and preparation methods and applications thereof, in which the substituents at positions 2, 6, 8, and 3 of coelenterazine are modified to obtain increased bioluminescence intensity or extended duration or bioluminescence wavelength with increased redshift or stability. Even though this document suggests that positions R3 and R4 can comprise an Electron Withdrawing Group, it is not disclosed that said Electron Withdrawing Group has organic spin converters.

### Summary

The present application discloses a chemiluminescent imidazopyrazinone-based photosensitizer compound, including salts, tautomers and stereoisomers thereof, with available singlet and triplet excited states of formula (I): 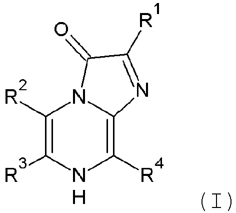 wherein,
R¹ is a hydrogen, an alkyl group, an amine group, a substituted or unsubstituted aryl, a substituted or unsubstituted heteroaryl or an Electron Donating Group;
R² is a hydrogen or an alkyl group;
R³ is a substituted or unsubstituted aryl, a substituted or unsubstituted heteroaryl that is an Electron Withdrawing Group with organic spin converters;
R⁴ is a halogen atom, a substituted or unsubstituted aryl, a substituted or unsubstituted heteroaryl that is an Electron Withdrawing Group with organic spin converters; wherein at least one of R³ or R⁴ is a substituted or unsubstituted heteroaryl that is an Electron Withdrawing Group with organic spin converters.

In one embodiment the aryl is selected from a group comprising phenyl, naphthyl, styryl and benzophenanthryl.

In another embodiment the heteroaryl is selected from a group comprising thienyl, thiazolyl, thiophenyl, furyl and pyrazolyl.

In yet another embodiment the substituted or unsubstituted aryl is selected from a group comprising phenyl, a naphthyl and hydroxyphenyl groups.

In another embodiment the Electron Withdrawing Group with organic spin converters is fullerene.

The present application also discloses a method to produce the compound of formula (I) comprising the reaction between compounds of formula (I-1) and compounds of formula (1-2): 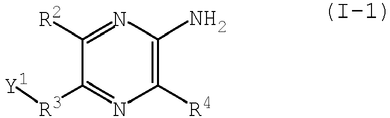 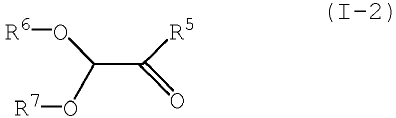 wherein R², R³ and R⁴ of I-1 are the same as described in claim 1 and Y¹ is a protecting group, and R⁵ of compounds of formula 1-2 is selected from a group comprising methyl, ethyl, propyl, butyl and isopropyl, and comprising the following steps:
- deprotection of 1-1;
- I-1 is dissolved in a solvent with 1-2 and kept under argon atmosphere;
- addition of a concentrated reagent;
- the mixture is heated and stirred;
- after cooling to a temperature between 20 and 25°C, the solvent is evaporated under reduced pressure;
- the residue is washed with AcOEt and ether.

In one embodiment R⁶ and R⁷ of compounds of formula 1-2 are combined to form an alkylene having 2 to 4 carbon atoms.

In another embodiment the Y¹ protecting group is selected from a group comprising tert-butyldimethylsilyl (TBDMS), trimethylsilyl (TMS), triethylsilyl (TES), triisopropylsilyl(TIPS), phenylsulfonyl and toluenesulfonyl (Tosyl).

In one embodiment the reagent is selected from a group comprising an acid catalyst such as hydrochloric acid, hydrobromic acid, hydroiodic acid, hydrofluoric acid, perchloric acid, sulfuric acid, phosphoric acid, nitric acid, acetic acid or mixtures thereof.

In yet another embodiment the solvent is selected from a group comprising dioxane, tetrahydrofuran, toluene, acetonitrile, acetone, N,N-dimethylformamide (DMF), dimethylsulfoxide (DMSO), ether, methanol, ethanol, water or mixtures thereof.

In one embodiment the reaction temperature is between 80°C to 180°C.

In another embodiment the reaction time is between 4 to 8 hours.

In one embodiment the present application also refers to compound of formula (I) for the use in photodynamic therapy of cancer.

In another embodiment the compound of formula (I) are used in cancer theranostics or diagnostics.

In yet another embodiment the compound of formula (I) is used in photocatalytic reactions.

### Disclosure

The present application discloses a self-activated chemiluminescent photosensitizer with available singlet and triplet excited states.

There are at least two main applications for this novel photosensitizer:
1 - Medical/pharmaceutical field, due to its use as a self-activated and tumor-selective photosensitizer for photodynamic therapy of cancer, which also emits light during treatment, thus allowing for tumor diagnostics and/or theranostics.
2 - Photocatalytic applications, due to its use as a self-activated photosensitizer with available singlet and triplet states, which can be used to carry out reactions at lower temperatures and more sustainable conditions. Photocatalysis can be applied on air/water purification, fuel production from wastewater or renewable feedstock and the synthesis of fine chemicals.

### 1) Medical/pharmaceutical applications:

Currently, clinical photodynamic therapy requires photosensitizers activated by external light/laser sources, that present several limitations, such as ineffectiveness, lack of selectivity and increased economic costs.

The technology disclosed herein solves the current limitations in clinical photodynamic therapy by developing a photosensitizer that can undergo self-excitation intracellularly, without the need for external light sources or any catalyst and co-factor, in a reaction with a cancer marker, such as overexpressed superoxide anion, thereby solving the problems associated with light-penetration in photodynamic therapy while maintaining the selectivity associated with photodynamic therapy. Moreover, as the developed photosensitizer is at the same time the photosensitizer and the excitation source, no energy transfer step is required. Also, no excitation equipment is now required, which decreases the cost and complexity of this therapy, while increasing its availability.

The new type of photosensitizer disclosed herein eliminates the restrictions that photodynamic therapy currently possesses regarding the size and localization of tumors, while maintaining the minimally-invasive character and limited side-effects. Moreover, the light-emitting properties of the photosensitizers also allow for real-time tumor diagnostic/imaging/theranostics.

For a successful cancer treatment, the combination of therapeutic with diagnostic/imaging is essential. However, imaging processes have the same problems as photodynamic therapy, as they also require light excitation by external light sources. Thus, the present technology of a chemiluminescent photosensitizer that can emit light and induce cytotoxicity at the same time allows for real-time cancer imaging/diagnostics irrespective of the size and localization of the tumor in the body. Moreover, no excitation equipment is needed, which decreases the cost and increases the availability of this diagnostic strategy. Finally, as no external excitation source is required, there is no autofluorescence and the background noise of emission is greatly decreased, which increases the sensitivity of the diagnostic.

### 2) Photocatalytic applications:

Photocatalysis is a subtype of catalysis and is based on the use of photons to drive chemical reactions, which enable obtaining several benefits. More specifically, the reactions can be carried out at lower temperatures than by using thermal energy and can be achieved higher selectivity for the formation of the desired products. In this field, catalysts are generally activated by light irradiation and undergo a transition to triplet excited states, which can be more easily involved on redox reactions needed to catalyze the target reactions directly. Moreover, triplet states are also able to interact with molecular oxygen to produce reactive oxygen species (such as singlet oxygen, hydroxyl radicals and hydrogen peroxide), which can act as reactive species that help to carry out the desired reaction. Photocatalysis has found widespread application in the fields of water splitting, CO₂ reduction, in the development of novel solar cells materials, in the synthesis of organic molecules, air/water purification, and fuel production from wastewater or renewable feedstock. However, the use of photocatalysis requires the use of energy-consuming and potentially-expensive and complex light sources, to activate the photocatalyst. Moreover, as the typical intersystem crossing rates of organic molecules is rather low, photocatalysts are generally based on metal elements (which possess higher intersystem crossing rates). However, the use of these elements increases the cost and potential toxicity of these approaches.

More specifically, we have the examples of the use of photocatalysts in the degradation of pollutants such as bisphenol A (Neamtu el al, Applied Catalysis B: Environmental 2018, 232, 553), possessing antimicrobial activity (Lanzilotto et al, JBIC Journal of Biological Inorganic Chemistry 2018, 23, 109), and in the synthesis of building blocks for polymers (Ciriminna et a, Chem Commun 2018, 54, 1008; Xu et al, Journal of the American Chemical Society 2017, 139, 41, 14775). These photocatalytic processes consist all on the use of different photocatalysts that can photosensitize the formation of singlet oxygen, which then drives the desired reaction. However, all the used photocatalysts required energy-consuming external light sources, and several of those processes required the presence of high cost/toxicity and low sustainability metal elements to allow for singlet oxygen photosensitization. The proposed technology consists on new photosensitizer compounds with readily available triplet states that can produce singlet oxygen, without the need for metal elements in their composition. This reduces the cost and toxicity compared to metal-based photocatalysts. Moreover, production of catalytic triplet states by the proposed photosensitizers can be activated without the need of external light sources, due to chemiluminescent reactions, which reduces the cost, complexity and energy-requirements of current photocatalysts.

These novel photosensitizer compounds allow for the self-excited production of singlet and/or triplet excited states, by undergoing a chemiluminescent reaction. This class of compounds reacts readily with molecular oxygen and reactive oxygen species to produce the target excited states, without the need for external light and laser activation. Moreover, the produced compounds are fluorescent molecules.

Also, the synthesis of these novel compounds is simple and straightforward, and no equipment is needed for inducing the chemiluminescent reaction. Thus, the developed compounds are ideal for application in photodynamic therapy and theranostics of cancer, in imaging and photocatalysis processes.

The novel compounds can act as tumor-selective chemiluminescent photosensitizers in photodynamic therapy of cancer, by inducing cytotoxicity due to an intracellular reaction with superoxide anion (which is overexpressed in tumor cells), without the need for light activation with optical and laser sources. This development will allow for the use of photodynamic therapy in tumors irrespective of their sizes and localization in the body. As the chemiluminescent substrate is also the photosensitizer, no energy transfer step between the excitation source and the photosensitizer is needed, thereby increasing efficiency of the process. As these compounds undergo chemiluminescence when reacting with superoxide anion, which is overexpressed in tumor cells, this system possesses an intrinsic tumorselectivity mechanism. Also, no other substrate/co-factor/catalyst is required.

With these advances, the use of photodynamic therapy can be expanded to all types of tumors, instead of just the ones on or just under the skin or on the lining of internal organs/cavities, which will make this therapy more relevant and more interesting to the pharmaceutical and medical sectors. Other factors that increase the value of this new modality is its minimally-invasive character and its lack of relevant side-effects, due to its selectivity. Moreover, the absence of expensive equipment as laser/light sources, will reduce the cost and complexity of the therapy.

Besides the induction of cytotoxicity, these chemiluminescent photosensitizers are also able to emit light during their reaction with superoxide anion (which is overexpressed in tumor cells), without the use of external light sources. This feature allows for the imaging of tumor cells, as well as its diagnostic. Thus, by combining a therapeutic and an imaging feature in the same molecule, these new compounds can be used in theranostic approaches.

The self-excited production of triplet excited states allows the use of the developed compound as catalysts in photocatalysis processes, without the use of energy-consuming and potentially-expensive light sources. Moreover, it allows the production of triplet excited states without the need for metal elements, which reduces the cost and potential toxicity of the photocatalytic approach.

Several features are described hereafter that can each be used independently of one another or with any combination of the other features. However, any individual feature might not address any of the problems discussed above or might only address one of the problems discussed above. Some of the problems discussed above might not be fully addressed by any of the features described herein. Although headings are provided, information related to a particular heading, but not found in the section having that heading, may also be found elsewhere in the specification.

### Brief description of drawings

For easier understanding of this application, figures are attached in the annex that represent the preferred forms of implementation which nevertheless are not intended to limit the technique disclosed herein.

The accompanying drawings illustrate various embodiments of the present application and are a part of the specification. The illustrated embodiments are merely examples of the present invention and do not limit the scope of the application.
Figure 1 illustrates structure of the imidazopyrazinone core.
Figure 2 illustrates structure of intermediate 1-1.
Figure 3 illustrates structure of intermediate 1-2.
Figure 4 illustrates structures of different photosensitizer compounds of formula (I).
Figure 5 illustrates the synthesis scheme of intermediate I-2 (2-Aminopyrazine derivatives) (15).
Figure 6 illustrates the synthesis scheme for 8-bromo-6-(4-hydroxyphenyl)-2-methylimidazo[1,2-a]pyrazin-3(7H)-one (1).
Figure 7 illustrates the synthesis scheme for 6-(4-hydroxyphenyl)-8-iodo-2-methylimidazo[1,2-a]pyrazin-3(7H)-one (2).
Figure 8 illustrates the synthesis scheme for 8-bromo-6-(1H-indol-3-yl)-2-methylimidazo[1,2-a]pyrazin-3(7H)-one (3).
Figure 9 illustrates the synthesis scheme for 6,8-dibromo-2-methylimidazo[1,2-a]pyrazin-3(7H)-one (4).
Figure 10 shows the measured chemiluminescence emission by compound 1.
Figure 11 shows the structure of compound 1 represented in figure 6.
Figure 12 shows the fluorescence intensity of ABDA in the presence of either compound 1 (concentration of 0-15 microMolar) or compound 1 and sodium azide.
Figure 13 shows the MTT reduction assays for assessing the cytotoxicity of compound 1 towards HepG2 tumor cell lines.

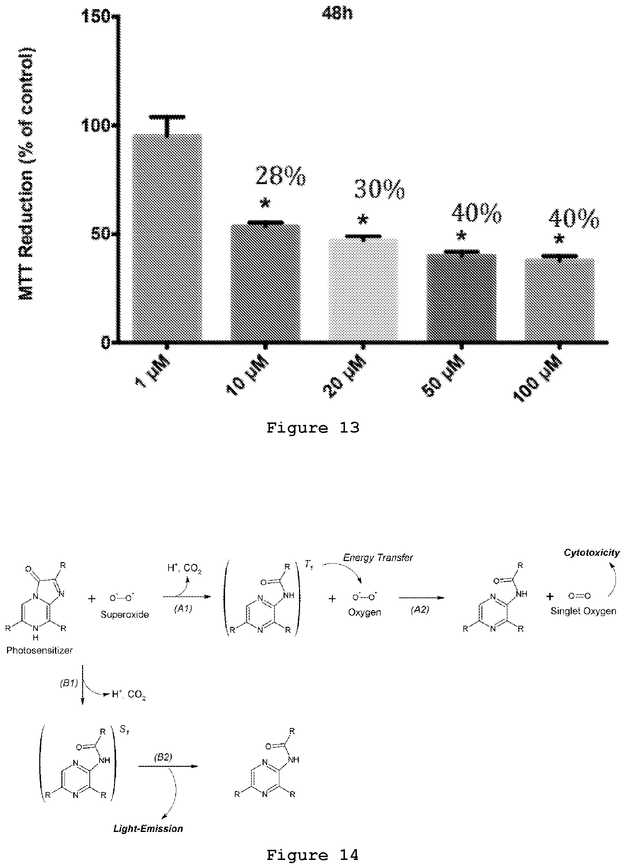

Figure 14 shows the mechanism for the production of triplet (T1) and singlet excited (S1) states for cancer photodynamic therapy and diagnostics/theranostics. The photosensitizer is oxidized selectively by superoxide anion (which can be considered as a cancer marker), generating T1 (pathway A1) and/or S1 (pathway B2) states. The T1-to-S1 ratio is modulated by the presence of spin converters functionalized on the imidazopyrazinone core. In the A pathway, the oxidized form of the photosensitizer transfers its energy to oxygen molecules, and produces the reactive species singlet oxygen, which is able to induce cytotoxicity on tumor cells. In the B pathway, the oxidized form of the photosensitizer emits light that can be detected/quantified by microscopic, spectroscopic and luminometric approaches, thereby allowing for cancer diagnostics/imaging/theranostics.

### Description of embodiments

Now, preferred embodiments of the present application will be described in detail with reference to the annexed drawings. However, they are not intended to limit the scope of this application.

The present application describes herein a chemiluminescent photosensitizer imidazopyrazinone core-based compound of formula (I) in detail. The presently described compounds of formula (I), also referred to as the "Imidazopyrazinone core-based molecule of the present application", also include salts, tautomers and stereoisomers thereof, or mixtures thereof in all ratios.

"Tautomers" refers to isomeric forms of a compound that are in equilibrium with each other. The concentrations of the isomeric forms will depend on the environment the compound is found in and may be different depending upon, for example, whether the compound is a solid or is in an organic or aqueous solution.

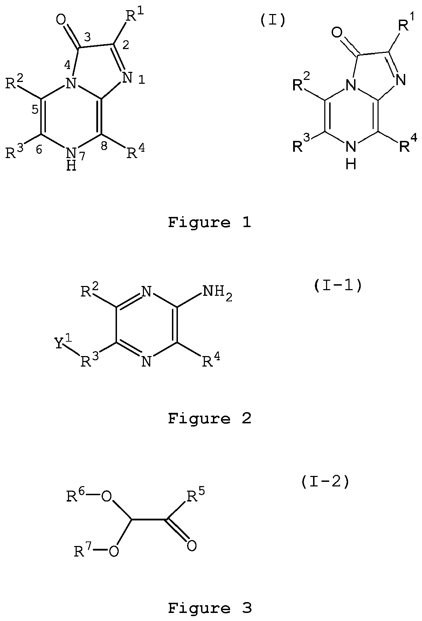

Imidazopyrazinone core-based molecules are modified at the C-2, C-5, C-6 and C-8 position of the structural core (Figure 1), wherein R¹ is an hydrogen, an alkyl group, an amine group, a substituted or unsubstituted aryl, a substituted or unsubstituted heteroaryl or an Electron Donating Group (EDG) , R² is a hydrogen or an alkyl group, R³ is a substituted or unsubstituted aryl, a substituted or unsubstituted heteroaryl that is an Electron Withdrawing Group (EWG) with organic spin converters, R⁴ is a halogen atom, a substituted or unsubstituted aryl, a substituted or unsubstituted heteroaryl that is an Electron Withdrawing Group (EWG) with organic spin converters.

The term "aryl" refers to a monovalent moiety obtained by removing a hydrogen atom from an aromatic ring. An aryl group may have from 6-10 carbon atoms (C₆₋₁₀ aryl) . In an embodiment the aryl group is phenyl, naphthyl, styryl and benzophenanthryl, among others, and their substituted and isomeric derivatives.

The term "heteroaryl" refers to a monovalent moiety obtained by removing a hydrogen atom from a heteroaromatic ring. In an embodiment, the ring atoms are carbon, nitrogen, sulfur or oxygen. More than one heteroatom may be present in the ring. In an embodiment the heteroaryl group is thienyl, thiazolyl, thiophenyl, furyl or pyrazolyl.

In a preferred embodiment, the "substituted or unsubstituted aryl" specifically includes phenyl, a naphthyl or a hydroxyphenyl group.

In a preferred embodiment, the "substituted or unsubstituted heteroaryl" is an indolylpyrazine, or an Electron Withdrawing Group (EWG) with organic spin converters. In an embodiment, the Electron Withdrawing Group (EWG) with organic spin converters is fullerene.

The present application also relates to a method for producing the Imidazopyrazinone core-based photosensitizer compounds of the present application.

Imidazopyrazinone core-based compound represented by the general formula (I) (wherein R¹, R², R³ and R⁴ are the same as defined above), is produced as follows:
- reacting a compound represented by general formula (I-1) found in Figure 2, (wherein R², R³ and R⁴ are the same as defined above, and Y¹ is a protecting group) with a compound represented by general formula (1-2) found in Figure 3 (R⁵ is independently methyl, ethyl, propyl, butyl or isopropyl. In one embodiment R⁶ and R⁷ are combined to form an alkylene having 2 to 4 carbon atoms). Thus, the compound represented by general formula (I) is obtained.

As used herein, the protecting group Y¹ independently represent tert-butyldimethylsilyl (TBDMS), trimethylsilyl (TMS), triethylsilyl (TES), triisopropylsilyl(TIPS), phenylsulfonyl, toluenesulfonyl (Tosyl), among others.

In a preferred embodiment the protecting groups are TBDMS or phenylsulphonyl.

The compound represented by general formula (I-1) can be produced by known methods (for example, by methods described in Y. Kishi et al., TetrahedronLett., 13,2747-2748 (1972), M. Adamczyk et al., Org. Prep. Proced. Int, 33,477-485 (2001)); or alternatively, the compound can be produced either by the Suzuki-Miyaura coupling reaction of a 2-amino-5-bromopyrazine derivative (F. D. Wael etal., Bioorg. Med. Chem., 17, 4336-4344 (2009), PCT /IB2010/053187, US 2011/0244481 Al).

The compound represented by general formula (I-2) can be produced by known methods (for example, the methods described in M. Adamczyk, M. et al., Synth. Commun., 32,3199-3205 (2002) or H. Baganz& H.-J. May, Chem. Ber, 99, 3766-3770 (1966) and H. Baganz & H.-J. May, Angew. Chem. Mt. Ed. Eng., 5, 420 (1966), or modifications of these methods).

The present application has the advantage that an optimized method for synthesizing Imidazopyrazinone core-based molecules (I) is provided. The compound represented by general formula (I-1) is synthesized through a four-step reaction according to a simple and convenient route based on halogenations and Suzuki coupling reactions.

The method steps to produce the compound of formula (I) are as follows:
The protected compound I-1 is deprotected before an acid treatment to react with compound 1-2. In detail, I-1 is dissolved in a solvent with 1-2 and kept under argon atmosphere. To the reaction mixture is added a concentrated reagent. The mixture is heated and stirred. After cooling to a temperature between 20 and 25°C, the solvent is evaporated under reduced pressure and the residue is washed with AcOEt and ether. The desired compound is obtained without any further purification.

The reagent used in the method for producing the compound of the present application represented by general formula (I) is an acid catalyst such as hydrochloric acid, hydrobromic acid, hydroiodic acid, hydrofluoric acid, perchloric acid, sulphuric acid, phosphoric acid, nitric acid, acetic acid, etc. These reagents may be used alone or as a mixture thereof.

In a particular embodiment the reagent is hydrochloric acid.

The solvent used in the method for producing the compound of general formula (I) is not particularly limited, but various solvents can be used. In one embodiment the solvent is dioxane, tetrahydrofuran, toluene, acetonitrile, acetone, N,N-dimethylformamide (DMF), dimethylsulfoxide (DMSO), ether, methanol, ethanol or water, etc., which can be used alone or as a mixture thereof. In a preferred embodiment the solvent is ethanol.

In the method for producing the compound of general formula(I), the reaction temperature and reaction time are not particularly limited. In an embodiment the temperature of the reaction ranges between 80°C to 180°C, and the time of the reaction ranges between 4 to 8 hours.

Herein, other advantage is that raw materials used in the method are cheap and readily available, the cost is low, experimental steps are simple, the yield of various steps is high, and the method is suitable for industrialized scaleup production.

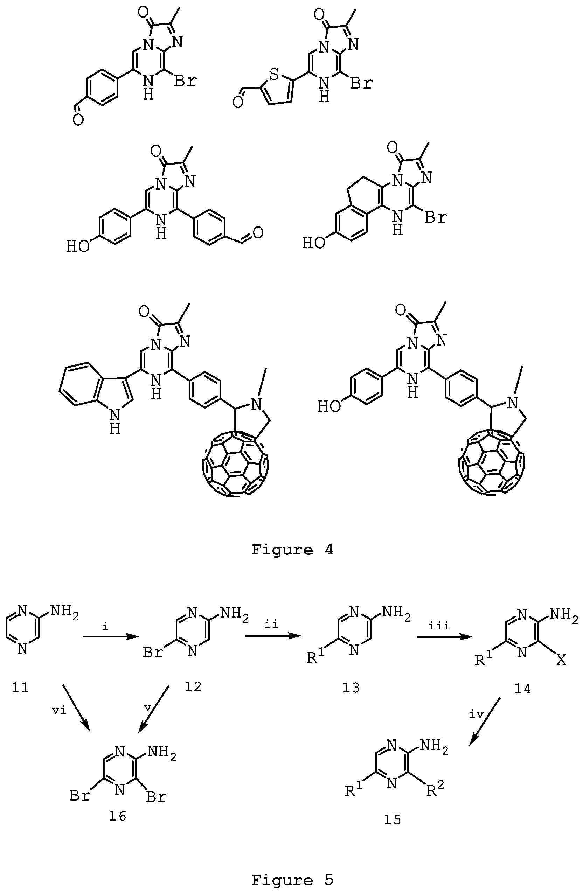

In a more preferred embodiment of the present application, imidazopyrazinone core-based compounds includes the different structures found on Figure 4.

The present application consists on new photosensitizers that are capable of intracellular self-excitation without needing external excitation sources (as laser and light sources), and of singlet oxygen sensitization by production of triplet excited states. In one embodiment, their trigger is the reaction with superoxide anion, which is overexpressed on tumor cells.

One of the objectives of the present application is the development of a tumor-selective photosensitizer that is both a chemiexcitation source and a photosensitizer, which can produce triplet excited states intracellularly and in a tumor-selective way, irrespective of the size and localization of the tumors.

By eliminating the need of external light, these new photosensitizers allow the minimally-invasive photodynamic therapy of cancer (which is known for its limited number of side-effects) to be used in any type of tumors, irrespective of their size and localization in the body. Nowadays, due to problems associated with light-penetration into the tissues, photodynamic therapy is only used for tumors on or just under the skin, or on the linings of internal organs and cavities. This technology eliminates the need for external excitation sources for activation, as well as energy transfer steps for the photosensitization of singlet oxygen and the production of triplet excited states. Moreover, the activation of these molecules does not require any catalyst or co-factor, as it can be activated by either oxygen or reactive oxygen species.

The process by which these photosensitizer molecules can be used on photodynamic therapy of cancer and cancer theranostics/diagnostics is detailed as follows (Figure 14): Upon administration to the cancer patient with the photosensitizer, and subsequent delivery of the photosensitizer to tumor cells, the photosensitizer reacts with the superoxide anion (which is overexpressed in tumor cells). This reaction oxidizes the imidazopyrazinone core and triggers the electronic transition from the fundamental state (S0) to the first singlet (S1) or triplet excited state (T1). The S1-T1 ratio is controlled by the spin converters present in the photosensitizer. The T1 molecules transfer their energy to molecular oxygen, thus forming singlet oxygen, which can induce the destruction of tumor cells by necrosis/apoptosis, vessels shutdown and immunostimulation. The S1 molecules decay to the S0 state with emission of visible light, which can be monitored by microscopic, spectroscopic and luminometric approaches, and allow for the real-time imaging of the therapeutic process. Moreover, as the photosensitizer and its oxidized counterpart have different fluorescent properties, their fluorescence can be further used in the tumor diagnostic.

It should be noted that the proposed therapeutic process consists on the exchange of superoxide anion with singlet oxygen. This approach is able to destroy cancer cells as superoxide is only a mild oxidant, with limited reactivity toward non-radical species. For the contrary, singlet oxygen is a strong oxidant with considerable reactivity towards important molecules as nucleic acids, proteins and lipids. Also, superoxide is one of the main targets of the cell antioxidant machinery, while singlet oxygen is not. Thus, this exchange should produce oxidative damage that is able to bypass the cell defenses.

The emission of light during the self-excitation reaction, and the resulting non-autofluorescence and low background noise, will allow for combining the therapeutic modality with tumor imaging/diagnostic, thereby leading to tumor theranostics. This will be achieved without complex and costly equipment for external excitation.

The possibility of producing triplet excited states without the use of energy-consuming light sources will allow for the use of these novel compounds as photocatalysts in watersplitting, CO₂ reduction, photovoltaics and organic synthesis, with reduced costs and without the need for excitation equipment. More specifically, when added to aprotic solvents, the developed compounds can interact with molecular oxygen dissolved in the solutions to produce reactive triplet excited states. These states transfer their energy to reactants/substrates, thereby selectively inducing the required chemical reactions. In protic solvents, the production of triplet states is possible by addition of superoxide anion production systems.

The presently disclosed technology comprises the following technical characteristics:
- The presence of spin converters (either at the C-6 or C-8 position) functionalized on the imidazopyrazinone core, which increases the triplet-to-singlet ratio;
- The imidazopyrazinone core, which reacts selectively with superoxide anion, to generate excited state products;
- The presence of chromophoric moieties functionalized on the imidazopyrazinone core (either at the C-6 and C-8 position), which allow to tune the fluorescence quantum yield and color of emitted light.

The presently disclosed technology can further comprise the following technical characteristics:
- Presence of different functional groups that allow for the coupling with other single molecules, different nanoparticles (as mesoporous silica nanoparticles, carbon dots, graphene quantum dots, and other carbon-based materials), proteins, antibodies, DNA and other biomacromolecules in the C-2 position.
- Presence of alkyl moieties in the C-2-position of the imidazopyrazinone core, which decreases the energy required for the reaction with superoxide anion.

### Examples:

### Synthesis Examples

2-Aminopyrazine derivatives (15) were synthesized following the procedure described in Figure 5.

Reagents: i) NBS, CH₂Cl₂ ii) R¹B(OH)₂, PdCl₂(PPh₃)₂, Na₂CO₃ 1M, toluene/EtOH iii) NXS, DMF iv) R²B(OH)₂, PdCl₂(PPh₃)₂, Na₂CO₃ 1M, toluene/EtOH v) NBS, CH₂Cl₂ vi)NBS, DMF 2-Aminopyrazine (11) was selectively brominated in position 3 with N-bromosuccinimide (NBS) in dichloromethane to obtain bromoderivative (12) used for the Suzuki coupling with the appropriate boronic acids, to obtain the derivatives (13). These compounds were then treated with NBS (or NIS) in DMF to afford a 3-bromo-aminopyrazine ready available (14) or for using in other Suzuki coupling (15).

5-bromopyrazin-2-amine (12) (Know compound, Reference: PCT /IB2010/053187) can be produced by known production methods or modification of these methods. Alternatively, the compound may also be commercially available.

To a cooled solution of 2-aminopyrazine (2g, 21mmol) in dichloromethane (50 mL) N-bromosuccinimide (NBS) (3.73g, 21mmol) was added in portion. The resulting mixture was stirred at 0°C for 2h and washed with saturated Na₂CO₃ solution and water.

The organic layer was dried over anhydrous Na₂SO₄, filtered and the solvent removed under reduced pressure to afford the crude product. The residue was purified by flash column chromatography (SiO₂, hexane/ethyl acetate 3/1 (v/v)) to yield the title compound as a light yellow solid. (2.88 g, 78.7%). ¹H-RMN (CDCl₃, 400 MHz) δ ppm: 8.09 (d, 1H), 7.77 (d, 1H), 4.60 (bs, 2H).

### General procedure of the Suzuki coupling for the synthesis of compounds (13):

A mixture of 2-amino-5-bromopyrazine (1 equiv) and an appropriate boronic acid (1.2-1.5 equiv) were dissolved in toluene (3 mL/mmol) and stirred at a temperature between 20 to 25°C. Ethanol (0.4 mL/mmol) and 1 M Na₂CO₃ aq. (1 equiv) were added to the reaction mixture and the resulting solution was degassed with Argon. Then, the catalyst bis-(triphenylphosphine) palladium (II) chloride (5.5%) in toluene was added in rapid sequence to the solution, and the mixture was deaerated again under argon atmosphere/argon gas protection. The resulting mixture was heated to reflux for 2-4h at 80-150°C depending on the boronic acid used (the progress of the reaction was monitored by TLC). After cooling to a temperature between 20 to 25°C, the solution was filtered through a Celite pad to remove the palladium catalyst. The solution was extracted with ethyl acetate, and the organic phase was washed with water and brine, dried over Na₂SO₄ and evaporated. The purification of the crude was done by flash chromatography (eluent reported in the compound description) to get the desired compound.

General procedure of the condensation of aminopyrazines (1-5) with glyoxal derivatives for the synthesis of compounds Aminopyrazin derivative (1 equiv) was dissolved in ethanol (3-5 mL/mmol) and kept under argon atmosphere. To the reaction mixture were added an appropriate glyoxal derivative (1.5-2 equiv) and concentrated hydrochloric acid (4-5 equiv). The mixture was stirred for 4-8 hours at 80°C to 120°C. After cooling to a temperature between 20 to 25°C, the solvent was evaporated under reduced pressure and the residue was washed with AcOEt and ether, affording the desired compound.

3,5-dibromopyrazin-2-amine (Know compound, Reference: R Jeanjot, et al., Synthesis, 513522 (2003).

2-amino-3,5-dibromopyrazine (16) can be produced by known production methods or modification of these methods. Alternatively, the compound may also be commercially available.¹H-RMN (CDCl₃, 400 MHz) δ ppm: 8.05 (s, 1H), 5.05 (s, 2H).

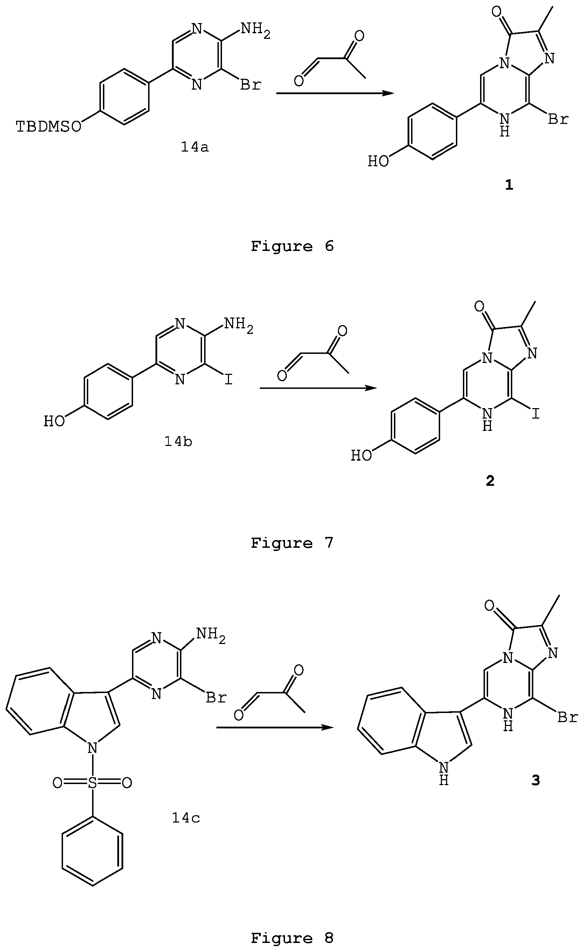

### Synthesis of 8-bromo-6-(4-hydroxyphenyl)-2-methylimidazo[1,2-a]pyrazin-3(7H)-one (1) (in Figure 6):

3-bromo-5-(4-((tert-butyldimethylsilyl)oxy)phenyl)pyrazin-2-amine (protected compound) was dissolved in THF at 0°C and TBAF was added dropwise into the solution and the mixture was stirred for 10 minutes. Then, the reaction mixture stirred at a temperature between 20 to 25°C for 3 hours. After that, the solution was poured into water extracted with ethyl acetate and dried over Na₂SO₄ anhydrous. The residue was purified by acid-base extraction affording 4-(5-amino-6-bromopyrazin-2-yl)phenol as the deprotected compound.

4-(5-amino-6-bromopyrazin-2-yl)phenol (1 equiv) was dissolved in ethanol (3 mL/mmol) and kept under argon atmosphere. To the reaction mixture were added methylglyoxal (2 equiv) and concentrated hydrochloric acid (5 equiv). The mixture was stirred for 4 hours at 80°C. After cooling to a temperature between 20 to 25°C, the solvent was evaporated under reduced pressure and the residue was washed with AcOEt and ether, affording Compound 1a as a yellow powder.¹H-RMN (MeOD, 400 MHz) δ ppm: 8.69 (s, 1H), 7.94 (d, 2H), 6.94 (d, 2H), 2.57 (s, 3H).

4-(tert-Butyldimethylsilyloxy)phenylboronic acid (1b)(known compound, Reference: P. Jeanjot, et al., Synthesis, 513-522 (2003), WO 2008013280) can be produced by known production methods. Alternatively, the compound may also be commercially available.

A solution of 4-hydroxyphenylboronic acid (1 g, 7.25 mmol), tert-butyldimethylsilyl chloride (3.28 g, 21.76 mmol) and imidazole (2.47 g, 36.3 mmol) in 12 mL DMF was stirred 17h at a temperature between 20 and 25°C. The reaction mixture was diluted with ethyl acetate, washed with saturated sodium hydrogen carbonate solution, dried over anhydrous sodium sulphate, and concentrated under reduced pressure. The resulting residue was purified by silica gel flash column chromatography (SiO₂, hexane/ethyl acetate 3/1 (v/v)) to thereby obtain the titled compound as a white solid. (1.33 g, 72.7%). ¹H-RMN (CDCl₃, 400 MHz) δ ppm: 8.11 (d, 2H), 6.95 (d, 2H), 1.01 (s, 9H), 0.25 (s, 6H) .

5-[4-(tert-Butyldimethylsilyloxy)phenyl]pyrazin-2amine (13a) (known compound, US 2011/0244481 Al) can be produced by known production methods. Alternatively, a new method is employed as described in "general procedures of the Suzuki coupling for the synthesis of compounds".

The resulting residue was purified by silica gel column chromatography using hexane/ethyl acetate 2/1, v/v, affording a yellow solid. ¹H-RMN (CDCl₃, 400 MHz) δ ppm: 8.39 (d, 1H), 8.03 (d, 1H), 7.74 (d, 2H), 6.91 (d, 2H), 4.61 (s, 2H), 1.00 (s, 9H), 0.22 (s, 6H).

3-Bromo-5-[4-(tert-butyldimethylsilyloxy)phenyl] pyrazin-2-amine (14a) (known compound, Reference: F. D. Wael, et al., Bioorg. Med. Chem., 17, 43364344 (2009); US 2011/0244481 Al) can be produced by known production methods. Alternatively, a new method is employed as described below:
To a cooled solution of 5-(4-((tert-butyldimethylsilyl)oxy)phenyl)pyrazin-2-amine (1 equiv) in DMF N-Bromosuccinimide (1 equiv) was added in apportion at 0°C and the resulting mixture was stirred at a temperature between 20 to 25°C for 2 h. The reaction solution was washed with water and extracted with ethyl acetate. The organic layer was dried over Na₂SO₄ anhydrous, filtered and the solvent was removed under reduced pressure. The crude product thus obtained was purified using silica gel column chromatography (hexane/ EtOAc = 5:1) .¹H-RMN (CDCl₃, 400 MHz) δ ppm: 8.33 (s, 1H), 7.74 (d, 2H), 6.90 (d, 2H), 4.99 (s, 2H), 0.99 (s, 9H), 0.22 (s, 6H).

### Synthesis of 6-(4-hydroxyphenyl)-8-iodo-2-methylimidazo[1,2-a]pyrazin-3(7H)-one (2) (can be found on Figure 7):

4-(5-amino-6-iodopyrazin-2-yl)phenol (1 equiv) was dissolved in ethanol (3 mL/mmol) and kept under argon atmosphere. To the reaction mixture were added methylglyoxal (2 equiv) and concentrated hydrochloric acid (5 equiv). The mixture was stirred for 4 hours at 80°C. After cooling to a temperature between 20 to 25°C, the solvent was evaporated under reduced pressure and the residue was washed with EtOAc and ether, affording Compound 2a as a yellow powder.¹H-RMN (MeOD, 400 MHz) δ ppm: 8.72 (s, 1H), 7.94 (d, 2H), 6.94 (d, 2H), 2.57 (s, 3H).

### 3-Iodo-5-[4-(tert-butyldimethylsilyloxy)phenyl] pyrazin-2-amine (14b) can be prepared as follows:

To a solution of 5-(4-((tert-butyldimethylsilyl)oxy)phenyl)pyrazin-2- amine (1d)(0.18 g, 0.60 mmol) and trifluoroacetic acid (54.9 µL, 0.72mmol) in DMF (2 mL) was added N-Iodosuccinimide (0.15 g, 0.66 mmol) in DMF. The resulting mixture was stirred at 80° for 3h. After aqueous workup, the organic phase was extracted with EtOAc and brine (100 mL), dried over Na₂SO₄ anhydrous, filtered and the solvent was removed under reduced pressure.

Crude product was purified by flash chromatography, isolating the desired compounds from a mixture of compounds with similar polarity. ¹H-RMN (CDCl₃, 400 MHz) δ ppm: 8.26 (s, 1H), 7.74 (d, 2H), 6.89 (d, 2H), 5.06 (s, 2H).

### Synthesis of 8-bromo-6-(1H-indol-3-yl)-2-methylimidazo[1,2-a]pyrazin-3(7H)-one (3) (found on figure 8):

Compound 3 was prepared according to general procedure. The compound was purified by flash chromatography using a mixture of hexane/EtOAc 1:1.

5-(1-(phenylsulfonyl)-1H-indol-3-yl)pyrazin-2-amine (13c) was preparing according to general procedure. The resulting residue was purified by silica gel column chromatography using hexane/ethyl acetate 2/1, v/v, affording 330 mg (72%) as a green solid.¹H-RMN (CDCl₃, 400 MHz) δ ppm: 8.43 (d, 1H), 8.15 (d, 1H), 8.08 (d, 1H), 8.05 (d, 1H), 7.96 (s, 1H), 7.93 (d, 2H), 7.54 (t, 1H), 7.44 (t, 2H), 7.34 (m, 2H), 4.62 (bs, 2H) .

3-bromo-5-(1-(phenylsulfonyl)-1H-indol-3-yl)pyrazin-2-amine (14c) was prepared according to procedure described above. The compound was purified by flash chromatography using a mixture of hexane/EtOAc 1:1. The crude product thus obtained was purified using silica gel column chromatography (hexane/ EtOAc = 3:1) as a yellow solid (73.6 mg, 30%). ¹H-RMN (CDCl₃, 400 MHz) δ ppm: 8.37 (s, 1H), 8.15 (d, 1H), 8.03 (d, 1H), 7.96 (s, 1H), 7.92 (d, 2H), 7.55 (t, 1H, H16), 7.45 (t, 2H), 7.36 (m, 2H), 5.14 (bs, 2H).

3-iodo-5-(1-(phenylsulfonyl)-1H-indol-3-yl)pyrazin-2-amine (14d) was prepared according to procedure described above. The compound was purified by flash chromatography using a mixture of hexane/EtOAc 1:1. The crude product thus obtained was purified using silica gel column chromatography (hexane/ EtOAc = 3:1) as a yellow solid (73.6 mg, 30%). ¹H-RMN (CDCl₃, 400 MHz) δ ppm: **¹H-RMN** (CDCl3, 400 MHz) δ ppm: 8.39 (s, 1H), 8.17 (d, 1H), 8.04 (d, 1H), 7.96 (s, 1H, H6), 7.92 (d, 2H), 7.55 (t, 1H), 7.45 (t, 2H), 7.36 (m, 2H), 5.13 (bs, 2H).

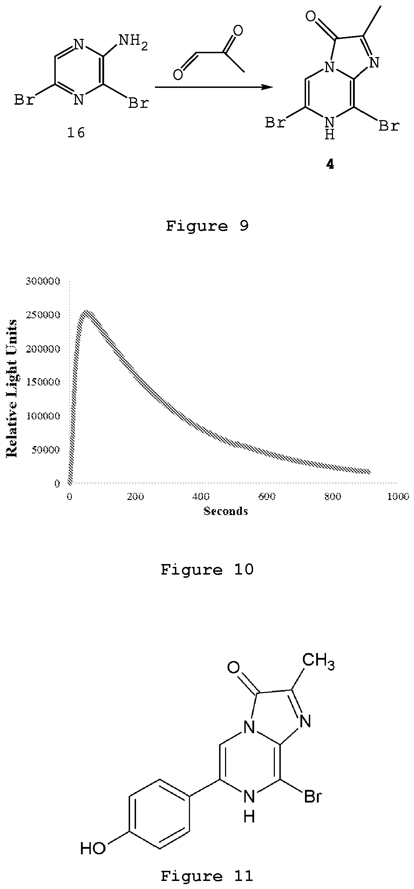

### Synthesis for 6,8-dibromo-2-methylimidazo[1,2-a]pyrazin-3(7H)-one (4) (found on Figure 9):

Compound 4 was prepared according to general procedure.¹H-RMN (MeOD, 400 MHz) δ ppm: 8.59 (d, 2H), 2.52 (s, 3H).

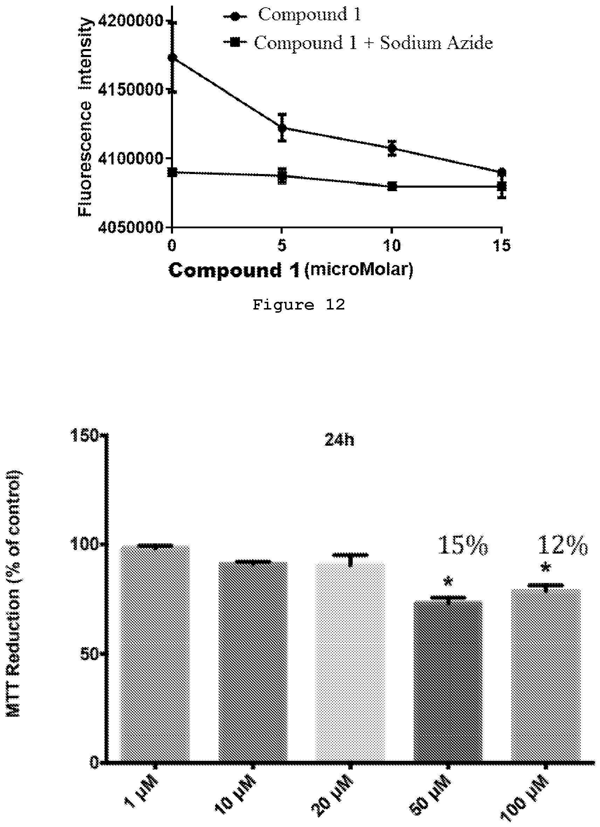

As an example, Figure 10 shows the light measured, as a function of time, from the chemiluminescent reaction of 8-bromo-6-(4-hydroxyphenyl)-2-methylimidazo[1,2-a]pyrazin-3(7H)-one (termed Compound 1, Figure 11). The light was measured in a homemade luminometer using a Hamamatsu HC135-01 photomultiplier tube. The chemiluminescent reaction took place at a temperature between 20 to 25°C, and were carried out in DMSO. The reaction was initiated by injection of the DMSO solution (400 µL) into an assay tube of compound 1 (30 µL) . The concentration of Compound 1 in the final solution was of 5 µM. The light was integrated and recorded in 0.1 s intervals. The production of light, without the use of an external excitation source, proves the formation of electronically-active singlet excited states available for bioimaging/diagnostics.

The production of reactive triplet states by compound 1 was determined by assessing the formation of singlet oxygen. This latter species is formed by energy transfer from a triplet molecule to oxygen. To this end, 9,10-anthracenediyl-bis(methylene)dimalonic acid (ABDA) was added to a solution containing compound 1, as the fluorescence of ABDA is quenched by the formation of singlet oxygen. Fluorescence was measured with a Horiba Jovin Yvon Fluoromax 4 spectrofluorometer, as seen in Figure 4. The fluorescence intensity of ABDA decreased significantly with increasing concentrations of compound, which supports the sensitization of singlet oxygen by compound 1. To confirm that the quenching of ABDA's fluorescence was due to the production of singlet oxygen, sodium azide was added to solutions containing ABDA and compound 1. Sodium azide is a known quencher of singlet oxygen. As expected, the addition of sodium azide prevented the quenching of the fluorescence emitted by ABDA (Figure 12).

To assess if the produced singlet oxygen can induce cytotoxicity in tumor cells, toxicity assays were performed *in vitro* with compound 1 in HepG2 human liver cancer cell lines. Cytotoxicity of compound 1 was evaluated through the MTT and LDH assay, which has been widely used as a colorimetric approach based on the activity of mitochondrial dehydrogenase enzyme in cells. MTT test was performed after an incubation of 24h and 48h. LDH release was monitored following an incubation of 24h and 48h with different concentrations of Compound 1 (1, 10, 20, 50 and 100 µM). Percent viability was obtained by comparing with control cell viability, considered as 100%. Data represents the mean ± SD of measurements of three independent experiments. Results of the one-way ANOVA factorial analysis. The results are shown in Figure 13. At 24h exposure, the concentrations of 50 and 100 µM showed a toxicity of 15% and 12% (respectively) in both MTT and LDH assays. At 48h exposure almost all the concentrations tested (with the exception of 1 µM) showed a considerable degree of toxicity, varying from 28% to 40% in the LDH assay. Thus, compound 1 was indeed able to induce cytotoxicity without the need of external excitation sources.

## Claims

1. Chemiluminescent imidazopyrazinone-based photosensitizer compound, including salts, tautomers and stereoisomers thereof, with available singlet and triplet excited states of formula (I): 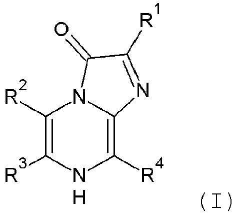 wherein,
R¹ is a hydrogen, an alkyl group, an amine group, a substituted or unsubstituted aryl, a substituted or unsubstituted heteroaryl or an Electron Donating Group;
R² is a hydrogen or an alkyl group;
R³ is a substituted or unsubstituted aryl, a substituted or unsubstituted heteroaryl that is an Electron Withdrawing Group with organic spin converters;
R⁴ is a halogen atom, a substituted or unsubstituted aryl, a substituted or unsubstituted heteroaryl that is an Electron Withdrawing Group with organic spin converters; wherein at least one of R³ or R⁴ is a substituted or unsubstituted heteroaryl that is an Electron Withdrawing Group with organic spin converters.

2. Compound of formula (I) according to claim 1, wherein the aryl is selected from a group comprising phenyl, naphthyl, styryl and benzophenanthryl.

3. Compound of formula (I) according to claim 1, wherein the heteroaryl is selected from a group comprising thienyl, thiazolyl, thiophenyl, furyl and pyrazolyl.

4. Compound of formula (I) according to claim 1, wherein the substituted or unsubstituted aryl is selected from a group comprising phenyl, a naphthyl and hydroxyphenyl groups.

5. Compound of formula (I) according to claim 1, wherein the Electron Withdrawing Group with organic spin converters is fullerene.

6. Method to produce the compound of formula (I) according to claim 1, comprising the reaction between compounds of formula (I-1) and compounds of formula (1-2): 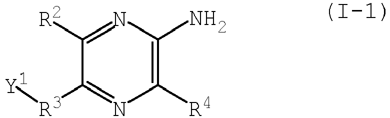 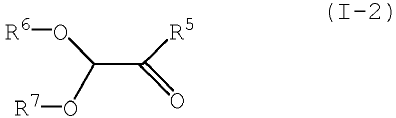
wherein R², R³ and R⁴ of I-1 are the same as described in claim 1 and Y¹ is a protecting group, and R⁵ of compounds of formula 1-2 is selected from a group comprising methyl, ethyl, propyl, butyl and isopropyl,
and comprising the following steps:
- deprotection of 1-1;
- I-1 is dissolved in a solvent with 1-2 and kept under argon atmosphere;
- addition of a concentrated reagent;
- the mixture is heated and stirred;
- after cooling to a temperature between 20 and 25°C, the solvent is evaporated under reduced pressure;
- the residue is washed with AcOEt and ether.

7. Method according to the previous claim, wherein R⁶ and R⁷ of compounds of formula 1-2 are combined to form an alkylene having 2 to 4 carbon atoms.

8. Method according to claim 6, wherein the Y¹ protecting group is selected from a group comprising tert-butyldimethylsilyl (TBDMS), trimethylsilyl (TMS), triethylsilyl (TES), triisopropylsilyl(TIPS), phenylsulfonyl and toluenesulfonyl (Tosyl).

9. Method according to any of the claims 6 to 8, wherein the reagent is selected from a group comprising an acid catalyst such as hydrochloric acid, hydrobromic acid, hydroiodic acid, hydrofluoric acid, perchloric acid, sulfuric acid, phosphoric acid, nitric acid, acetic acid or mixtures thereof.

10. Method according to any of the claims 6 to 9, wherein the solvent is selected from a group comprising dioxane, tetrahydrofuran, toluene, acetonitrile, acetone, N,N-dimethylformamide (DMF), dimethylsulfoxide (DMSO), ether, methanol, ethanol, water or mixtures thereof.

11. Method according to any of the claims 6 to 10, wherein the reaction temperature is between 80°C to 180°C.

12. Method according to any of the claims 6 to 11, wherein the reaction time is between 4 to 8 hours.

13. Compound of formula (I) according to claim 1, for the use in photodynamic therapy of cancer.

14. Compound of formula (I) according to claim 1, for the use in cancer theranostics or diagnostics.

15. Compound of formula (I) according to claim 1, for the use in photocatalytic reactions.

## Patentansprüche

1. Chemilumineszierende, auf Imidazopyrazinon basierte Photosensibilisator-Verbindung, einschließlich deren Salze, Tautomere und Singulett- und Triplett-Stereoisomere, mit verfügbaren angeregten Formelzuständen (I): 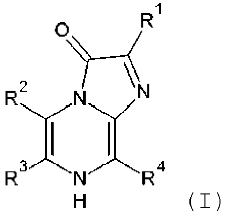 worin,
R¹ ein Wasserstoff, eine Alkylgruppe , eine Amingruppe, ein substituiertes oder unsubstituiertes Aryl, ein substituiertes oder unsubstituiertes Heteroaryl oder eine elektronenspendende Gruppe ist;
R² eine Wasserstoff- oder eine Alkylgruppe ist;
R³ ein substituiertes oder unsubstituiertes Aryl, ein substituiertes oder unsubstituiertes Heteroaryl ist, welches eine elektronenentziehende Gruppe mit organischen Spin-Konvertern darstellt;
R⁴ ein Halogenatom, ein substituiertes oder unsubstituiertes Aryl, ein substituiertes oder unsubstituiertes Heteroaryl ist, welches eine elektronenentziehende Gruppe mit organischen Spin-Konvertern ist; worin mindestens eines der R³ oder R⁴ Elemente ein substituiertes oder unsubstituiertes Heteroaryl ist, welches eine elektronenentziehende Gruppe mit organischen Spin-Konvertern darstellt;

2. Verbindung der Formel (I) gemäß Anspruch 1, worin das Aryl aus einer Gruppe ausgewählt wird, die aus Phenyl, Naphthyl, Styryl und Benzophenanthryl besteht.

3. Verbindung der Formel (I) gemäß Anspruch 1, worin das Heteroaryl aus einer Gruppe ausgewählt wird, die aus Thienyl, Thiazolyl, Thiophenyl, Furyl und Pyrazolyl besteht.

4. Verbindung der Formel (I) gemäß Anspruch 1, worin das substituierte oder unsubstituierte Aryl aus einer Gruppe ausgewählt wird, die Phenyl, ein Naphthyl und Hydroxyphenylgruppen enthält.

5. Verbindung der Formel (I) gemäß Anspruch 1, worin die elektronenentziehende Gruppe mit organischen Spin-Konvertern ein Fulleren ist.

6. Methode zur Herstellung der Verbindung von Formel (I) gemäß Anspruch 1, bestehend aus der Reaktion zwischen den Verbindungen der Formel (I-1) und Verbindungen der Formel (I-2) : 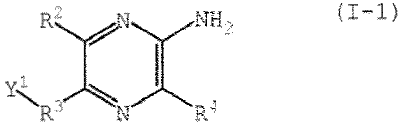 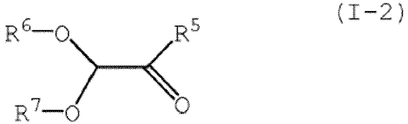 Worin R², R³ und R⁴ von Formel I-1 die gleichen sind wie in Anspruch 1 beschrieben wird und Y¹ eine Schutzgruppe ist, wobei R5 der Verbindungen von Formel I-2 aus einer Gruppe ausgewählt wird, die aus Methyl, Ethyl, Propyl, Butyl und Isopropyl besteht und die folgenden Schritte umfasst:
- Entschützung von I-1;
- I-1 wird mit I-2 in einem Lösungsmittel gelöst und unter Argon-Atmosphäre aufbewahrt;
- Zugabe eines konzentrierten Reagens;
- Erhitzen und Rühren der Mischung;
- Verdampfen des Lösungsmittels unter reduziertem Druck nach dem Abkühlen auf eine Temperatur zwischen 20 und 25 Grad Celsius;
- Waschen der Rückstände mit AcOEt und Äther.

7. Methode gemäß dem vorherigen Anspruch, worin R⁶ und R⁷ der Verbindungen von Formel I-2 zur Bildung eines Alkylens mit 2 bis 4 Kohlenstoffatomen kombiniert werden.

8. Methode gemäß Anspruch 6, worin die Y¹ -Schutzgruppe aus einer Gruppe ausgewählt wird, die Tert-Butyldimethylsilyl (TBDMS), Trimethylsilyl (TMS), Triethylsilyl (TES), Triisopropylsilyl (TIPS), Phenylsulfonyl und Toluolsulfonyl (Tosyl) enthält.

9. Methode gemäß einem der Ansprüche 6 bis 8, worin der Reagenzstoffe aus einer Gruppe ausgewählt ist, die einen Saeurekatalysator, wie Salzsäure, Bromwasserstoffsäure, Jodwasserstoffsäure, Flusssäure, Perchlorsäure, Schwefelsäure, Phosphorsäure, Salpetersäure, Essigsäure oder Gemischen davon enthält.

10. Methode gemäß einem der Ansprüche 6 bis 9, worin das Lösungsmittel aus einer Gruppe ausgewählt wird, die aus Dioxan, Tetrahydrofuran, Toluol, Acetonitril, Aceton, N,N-Dimethylformamid (DMF), Dimethylsulfoxid (DMSO), Äther, Methanol, Ethanol, Wasser oder Mischungen davon besteht.

11. Methode gemäß einem der Ansprüche 6 bis 10, worin die Reaktionstemperatur zwischen 80 und 180 Grad liegt.

12. Methode gemäß einem der Ansprüche 6 bis 11, worin die Reaktionszeit zwischen 4 und 8 Stunden liegt.

13. Verbindung von Formel (I) gemäß Anspruch 1, zur Verwendung bei der photodynamischen Therapie von Krebs.

14. Verbindung von Formel (I) gemäß Anspruch 1, zur Verwendung bei der Krebs-Theranostik oder Diagnostik.

15. Verbindung von Formel (I) gemäß Anspruch 1, zur Verwendung bei photokatalytischen Reaktionen.

## Revendications

1. Composé photosensibilisateur à base d'imidazopyrazinone, comprenant des sels, des tautomères et des stéréoisomères dans celui-ci, ayant des états excités singulet et triplet disponibles de formule (I) : 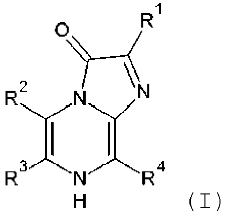 dans lequel,
R¹ est un hydrogène, un groupe alkyle, un groupe amine, un aryle substitué ou non substitué, un hétéroaryle substitué ou non substitué ou un groupe donneur d'électrons ;
R² est un hydrogène ou un groupe alkyle ;
R³ est un aryle substitué ou non substitué, un hétéroaryle substitué ou non substitué qui est un groupe attracteur d'électrons avec des convertisseurs de spin organiques ;
R⁴ est un atome d'halogène, un aryle substitué ou non substitué, un hétéroaryle substitué ou non substitué qui est un groupe attracteur d'électrons avec des convertisseurs de spin organiques ; dans lequel au moins un parmi R³ ou R⁴ est un hétéroaryle substitué ou non substitué qui est un groupe attracteur d'électrons avec des convertisseurs de spin organiques.

2. Composé de formule (I) selon la revendication 1, dans lequel l'aryle est sélectionné parmi un groupe comprenant le phényle, le naphthyle, le styryle et le benzophénanthryle.

3. Composé de formule (I) selon la revendication 1, dans lequel l'hétéroaryle est sélectionné parmi un groupe comprenant le thiényle, le thiazolyle, le thiophényle, le furyle et le pyrazolyle.

4. Composé de formule (I) selon la revendication 1, dans lequel l'aryle substitué ou non substitué est sélectionné parmi un groupe comprenant le phényle, un naphtyle et des groupes hydroxyphényles.

5. Composé de formule (I) selon la revendication 1, dans lequel le groupe attracteur d'électrons avec des convertisseurs de spin organiques est un fullerène.

6. Procédé pour produire le composé de formule (I) selon la revendication 1, comprenant la réaction entre des composés de formule (I-1) et des composés de formule (I-2) : 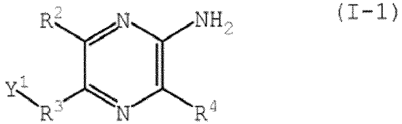 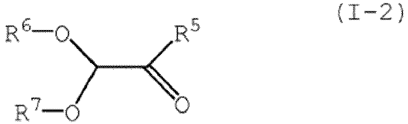 dans lequel R², R³ et R⁴ de I-1 sont les mêmes tels que décrits dans la revendication 1 et Y¹ est un groupe protecteur, et R⁵ des composés de formule I-2 est sélectionné parmi un groupe comprenant le méthyle, l'éthyle, le propyle, le butyle et l'isopropyle, et comprenant les étapes suivantes :
- déprotection de I-1 ;
- I-1 est dissout dans un solvant avec I-2 et conservé sous atmosphère d'argon ;
- ajout d'un réactif concentré ;
- le mélange est chauffé et remué ;
- après le refroidissement à une température entre 20 et 25 °C, le solvant est évaporé sous pression réduite ;
- le résidu est nettoyé avec de l'AcOEt et de l'éther.

7. Procédé selon la revendication précédente, dans lequel R⁶ et R⁷ des composés de formule I-2 sont combinés pour former un alkylène ayant de 2 à 4 atomes de carbone.

8. Procédé selon la revendication 6, dans lequel le groupe protecteur Y¹ est sélectionné parmi un groupe comprenant le tert-butyldiméthylsilyle (TBDMS), le triméthylsilyle (TMS), le triéthylsilyle (TES), le triisopropylsilyle (TIPS), le phénylsulfonyle et le toluènesulfonyle (Tosyle).

9. Procédé selon l'une quelconque des revendications 6 à 8, dans lequel le réactif est sélectionné parmi un groupe comprenant un catalyseur acide tel que l'acide chlorhydrique, l'acide bromhydrique, l'acide iodhydrique, l'acide fluorhydrique, l'acide perchlorique, l'acide sulfurique, l'acide phosphorique, l'acide nitrique, l'acide acétique ou leurs mélanges.

10. Procédé selon l'une quelconque des revendications 6 à 9, dans lequel le solvant est sélectionné parmi un groupe comprenant le dioxane, le tétrahydrofurane, le toluène, l'acétonitrile, l'acétone, le N,N-diméthylformamide (DMF), le diméthylsulfoxyde (DMSO), l'éther, le méthanol, l'éthanol, l'eau ou leurs mélanges.

11. Procédé selon l'une quelconque des revendications 6 à 10, dans lequel la température de réaction est comprise entre 80 °C et 180 °C.

12. Procédé selon l'une quelconque des revendications 6 à 11, dans lequel le temps de réaction est compris entre 4 et 8 heures.

13. Composé de formule (I) selon la revendication 1, pour l'utilisation en thérapie photodynamique du cancer.

14. Composé de formule (I) selon la revendication 1, pour l'utilisation en théranostique ou diagnostic du cancer.

15. Composé de formule (I) selon la revendication 1, pour l'utilisation en réactions photocatalytiques.
